# EUROPÄISCHE PATENTSCHRIFT

(11) **EP 2 997 983 B1**
(45) Veröffentlichungstag und Bekanntmachung des Hinweises auf die Patenterteilung: **09.05.2018**
(21) Anmeldenummer: 15185680.4
(22) Anmeldetag: 17.09.2015
(51) Int. Cl.: A61L 27/16, A61L 27/54, A61L 24/00, A61L 24/06

(54) **VERFAHREN ZUR HERSTELLUNG EINES ANTIBIOTISCHEN POLYMETHYLMETHACRYLAT-KNOCHENZEMENTPULVERS UND EIN ANTIBIOTISCHES POLYMETHYLMETHACRYLAT-KNOCHENZEMENTPULVER**
METHOD FOR PRODUCING AN ANTIOBIOTIC POLYMETHYLMETHACRYLATE BONE CEMENT POWDER, AND AN ANTIBIOTIC POLYMETHYLMETHACRYLATE BONE CEMENT POWDER
PROCEDE DE FABRICATION D'UNE POUDRE DE CIMENT OSSEUX EN POLYMETHACRYLATE DE METHYLE ANTIBIOTIQUE ET POUDRE DE CIMENT OSSEUX EN POLYMETHACRYLATE DE METHYLE ANTIBIOTIQUE

(30) Priorität: 19.09.2014 DE 102014218913
(43) Veröffentlichungstag der Anmeldung: 23.03.2016
(73) Patentinhaber: Heraeus Medical GmbH, 61273 Wehrheim (DE)
(72) Erfinder: Vogt, Sebastian, 99092 Erfurt (DE)
(74) Vertreter: Heraeus IP

(56) Entgegenhaltungen:
- EP-A2- 1 949 918

## Beschreibung

Gegenstand der Erfindung sind ein Verfahren zur Herstellung eines antibiotischen Knochenzementpulvers sowie das durch das Verfahren hergestellte antibiotische Knochenzementpulver.

Gelenkendoprothesen werden in großem Umfang bei unterschiedlichsten Gelenkerkrankungen mit sehr großem Erfolg zur Erhaltung der Bewegungsfunktionen der Patienten eingesetzt.
Leider kommt es bei einem geringen Anteil der Patienten zu Infektionen an den Gelenkendoprothesen und dem umgebenden Knochen- und Weichgewebe. Sehr häufig werden zur Behandlung dieser Infektionen der einzeitige und auch der zweizeitige Gelenkendoprothesenwechsel durchgeführt.

Für die dauerhafte mechanische Fixierung der Revisions-Gelenkendoprothesen haben sich Revisions-Polymethylmethacrylat-Knochenzemente bewährt, die ein Antibiotikum oder zwei oder auch mehrere Antibiotika enthalten. Durch diese Antibiotika wird die Revisions-Gelenkendoprothese und das umgebende Knochen- und Weichgewebe zu mindestens unmittelbar postoperativ vor einer erneuten mikrobiellen Besiedlung geschützt. Neben der individuellen Beimischung von Antibiotika durch den Arzt haben sich industriell hergestellte Revisons-Polymethylmethacrylat-Knochenzemente bewährt.

So werden von der Heraeus Medical GmbH die Revisions-Polymethylmethacrylat-Knochenzemente Copal® G+C und Copal® G+V hergestellt und vertrieben. Copal® G+C enthält die Kombination Gentamicin und Clindamycin. Copal® G+V enthält die Antibiotika Gentamicin und Vancomycin. Die Kombination Gentamicin mit Vancomycin eignet sich bisher besonders dann, wenn die Infektion der Gelenkendoprothese durch Methicillin-resistente Staphylokokken (MRSA, MRSE) hervorgerufen wird.

In EP 1 949 918 A2 ist ein Revisions-Polymethylmethacrylat-Knochenzement beschrieben. Dieser Knochenzement enthält eine Pulverkomponente mit zwei oder mehr granulären Antibiotika. Die Antibiotika stimmen in ihrer Korngrößenverteilung darin überein, dass jeweils die Haupt-Siebfraktion der einzelnen Antibiotika in ein- und demselben Korngrößenbereich liegt. Dadurch erfolgt eine hohe initiale Freisetzung aller im Zement enthaltenen Antibiotika erfolgt.

Seit einigen Jahren sind jedoch auch Vancomycin-resistente Staphylokokken- und Enterokokken-Stämme bekannt. Es ist damit zu rechnen, dass diese Vancomycin-resistenten Bakterien in den nächsten Jahren eine zunehmende Rolle als Verursacher von Gelenkassoziierten Infektionen spielen werden. Daher ist es sinnvoll, einen Revisions-Polymethylmethacrylat-Knochenzement zu entwickeln, der mindestens ein Antibiotikum enthält, das wirksam gegenüber Vancomycin-resistenten Bakterien ist. Daneben spielen auch zunehmend problematische Gram-negative Bakterien eine Rolle als Verursacher von Gelenkassoziierten Infektionen. Es handelt sich dabei insbesondere um sogenannte ESBL-Stämme.

Ein Antibiotikum mit einem sehr breiten Wirkungsspektrum stellt Fosfomycin dar. Das Antibiotikum Fosfomycin ((2*R*,3*S*)-3-Methyloxiranphosphonsäure, CAS 23155-02-4) wurde 1969 entdeckt (D. Hendlin et al.: Phosphonomycin a new antibiotic produced by strains of Streptomyces. Science 96 (1969) 122-123.)

Fosfomycin hemmt das bakterielle Enzym MurA (UDP-*N*-Acetylglucosamin-enolpyruvyltransferase) (F. M. Kahan et al.: The mechanism of action of fosfomycin (phosphonomycin). Ann N Y Acad Sci 235 (1974) 364-386.; E. D. Brown et al.: "MurA (MurZ), the enzyme that catalyzes the first committed step in peptidoglycan biosynthesis, is essential in Escherichia coli. J. Bacteriol. 177 (14) (1995) 4194-4197). Dieses katalysiert den ersten Schritt der Mureinbiosynthese. Bei diesem Schritt wird auf die Hydroxylgruppe an Position 3 des UDP-*N-*Acetylglucosamins eine Enolpyruvyleinheit ausgehend von Phosphoenolpyruvat (PEP) transferiert. Das bedeutet, es entsteht ein Milchsäureether an der Position 3 des UDP-*N-*Acetylglucosamins. Bei Störung dieses Schritts durch Fosfomycin wird die bakterielle Zellwandsynthese inhibiert.

Fosfomycin wirkt bei sensiblen Bakterien bakterizid. Fosfomycin erfasst Gram-negative und Gram-positive Bakterien, wobei auch Methicillin-resistente Staphylokokken eingeschlossen sind (W. Graninger et al.: In vitro activity of fosfomycin against methicillin-susceptible and methicillinresistant Staphylococcus aureus. Infection 12 (1984) 293-295). Fosfomycin ist auch bei Vancomycin-resistenten Staphylokokkus aureus (VRS) und auch bei Vancomycin-resistenten Enterokokken wirksam (F. Allerberger, I. Klare: In-vitro activity of fosfomycin against vancomycin-resistant enterococci. J Antimicrob Chemother 43 (1999) 211-217; T. Hara et al.: Antimicrobial activity of fosfoymcin against beta-lactamase-producing methicillin-sensitive Staphylococcus aureus and methicillin sensitive coagulase-negative staphylococci. Jpn J Antibiot 56 (20013) 142-147). Daneben ist Fosfomycin auch bei ESBL wirksam (M. E. Falagas et al.: Fosfomycin for the treatment of multidrug-resistant, including extended-spectrum ßlactamase producing enterobacteriaceae infections: a systematic review. Lancet Infect Dis 10 (2010) 43-50.).

Für die pharmazeutische Verwendung wird Fosfomycin in Salze überführt, die hinreichend lagerstabil sind und deren wässrige Lösungen einen physiologisch tolerierbaren pH-Wert haben. In der europäischen Pharmakopoe werden drei Salze des Fosfomycins beschrieben. Es handelt sich dabei um das Monohydrat des Calciumsalzes des Fosfomycins (CAS 26016-98-8), um das Dinatriumsalz des Fosfomycins (CAS 26016-99-9) und um Trometamol-Fosfomycin (CAS 78964-85-9).

Die Salze des Fosfomycins sind außerordentlich hygroskopisch. Sie ziehen Luftfeuchtigkeit an und zerfließen dabei. Versuche zeigten, dass die Salze im trockenen Zustand in Zementpulver von Polymethylmethacrylat-Knochenzementen integriert werden können. Aber die Knochenzementpulver ziehen bei Lagerung an Luft ebenfalls Luftfeuchtigkeit, wobei die Antibiotika-Partikel auch zerfließen. Dabei kann es zur Verklumpung des Knochenzementpulvers kommen. Daher sind die Salze des Fosfomycins für die industrielle Herstellung von antibiotischen Polymethylmethacrylat-Knochenzementpulvern nur sehr bedingt geeignet.

Die Aufgabe der Erfindung besteht darin, die vorstehend genannten Nachteile des Standes der Technik zu überwinden. Insbesondere besteht die Aufgabe der Erfindung darin, ein geeignetes Verfahren für die Herstellung eines fließ- und rieselfähigen Knochenzementpulvers zu entwickeln, das Fosfomycin enthält. Das Knochenzementpulver soll keine Klumpen oder sonstige Aggregate enthalten und soll als fließ- und rieselfähiges Pulver für die medizinische Anwendung zur Verfügung stehen.

Die Aufgabe der Erfindung wurde durch ein Verfahren zur Herstellung eines antibiotischen Knochenzementpulvers gelöst, wobei in einem Schritt A) ein Knochenzementbasispulver mit einem Wassergehalt kleiner gleich 1,0 Gew.-% mit Trometamol-Fosfomycin zu einem Knochenzementpulver vermischt wird und das Knochenzementpulver auf einen Wassergehalt von kleiner gleich 1,0 Gew.% getrocknet wird. Außerdem wird die Aufgabe der Erfindung durch ein Knochenzementpulver gelöst. Die Erfindung betrifft außerdem ein Knochenzementpulver, das nach einem solchen Verfahren hergestellt wurde und ein Knochenzementbasispulver und Trometamol-Fosfomycin enthält, wobei das Knochenzementpulver einen Wassergehalt von kleiner gleich 1,0 Gew.% hat.

Unter Knochenzementbasispulver sind erfindungsgemäß organische und anorganische Knochenersatzrohstoffe in Pulverform zu verstehen. Anorganische Knochenersatzrohstoffe sind beispielsweise Calciumphosphat oder Calciumsulfat, während es sich bei organischen Knochenersatzmaterialien um Polymere, insbesondere Acrylatpolymere handelt. Das Polymer kann ein Homopolymer oder ein Copolymer sein. Bevorzugt handelt es sich bei dem Knochenzementbasispulver um ein Polymer oder Copolymer eines Methacrylsäureesters in Pulverform. Gemäß einer besonders bevorzugten Ausführungsform ist das Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat), Poly(styren-co-methylmeth-acrylat) und einer Mischung aus mindestens zwei dieser Polymere besteht.

Das Knochenzementbasispulver wird dem Knochenzementpulver bevorzugt in einer Menge von 70 bis 99,5 Gew.%, besonders bevorzugt von 80,0 bis 94 Gew.-% zugemischt.

Die Erfindung basiert auf der überraschenden Beobachtung, dass mit dem erfindungsgemäßen Verfahren bei der Kombination von Trometamol-Fosfomycin mit an sich bekannten Knochenzementrohstoffen, insbesondere Polymethylmethacrylat-Knochenzementrohstoffen in Pulverform, ein nicht verklumpendes, lagerfähiges Knochenzementpulver hergestellt werden kann.

Bevorzugt wird dem Knochenzementpulver außerdem ein Polymerisationsinitiator beigemischt. Bei dem Polymerisationsinitiator handelt es sich vorzugsweise um einen aktivierbaren Polymerisationsinitiator, z. B. Peroxide und Barbitursäurederivate.

Unter einem Peroxid werden erfindungsgemäß Verbindungen verstanden, die wenigstens eine Peroxogruppe (-O-O-)enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel Dialkylperoxide oder Hydroperoxide. Beispielsweise kann das Peroxid aus der Gruppe ausgewählt sein, die aus Dibenzoylperoxid, Cumol-hydroperoxid, 1,1,3,3-Tetramethylbutyl-hydroperoxid, t-Butyl-hydroperoxid, t-Amyl-hydroperoxid, Di-Isopropylbenzen-mono-hydroperoxid und einer Mischung aus mindestens zwei davon besteht. Gemäß einer bevorzugten Ausführungsform ist das Peroxid aus der Gruppe ausgewählt, die aus Dibenzoylperoxid und Dilauroylperoxid besteht. Besonders bevorzugt ist mit Wasser phlegmatisiertes Dibenzoylperoxid mit einem Wassergehalt von weniger als 30 Gew.-%, bevorzugt 28 Gew..-%.

Bei dem Barbitursäurederivat handelt es sich z.B. um ein Barbitursäurederivat ausgewählt aus der Gruppe bestehend aus 1-monosubstituierten Barbituraten, 5-monosubstituierten Barbituraten, 1,5-disubstituierten Barbituraten- und 1,3,5-trisubstituierten Barbituraten. Bevorzugt ist das Barbitursäurederivat ausgewählt aus der Gruppe bestehend aus 1,5-disubstituierten Barbituraten- und 1,3,5-trisubstituierten Barbituraten. Die Art der Substituenten an der Barbitursäure ist nicht weiter eingeschränkt. Bei den Substituenten kann es sich beispielsweise um aliphatische oder aromatische Substituenten handeln. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethylbarbitursäure und 1,3,5-Trimethyl-barbitursäure besteht.

Denkbar ist auch ein Photoinitiator oder Photoiniatorsystem. Zusätzlich kann neben dem aktivierbaren Polymerisationsinitiator auch ein elektrisch leitfähiger Röntgenopaker zugemischt werden. Hier eignen sich besonders Partikel von Kobalt, Eisen, NdFeB, SmCo, Kobalt-ChromStahl, Zirkonium, Hafnium, Titan, Titan-Aluminium-Silizium-Legierungen und Titan-Niobium-Legierungen mit einer Partikelgröße von 0,5-500 µm. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Vorzugsweise wird dem Knochenzementpulver der Polymerisationsinitiator in einer Menge in einem Bereich von 0,01 bis 10 Gew.-%, mehr bevorzugt in einem Bereich von 0,2 bis 8 Gew.-% und noch mehr bevorzugt in einem Bereich von 0,5 bis 5 Gew.-%, jeweils bezogen auf das Gesamtgewicht des Knochenzementpulvers zugemischt.

Gemäß einer bevorzugten Ausführungsform des erfindungsgemäßen Verfahrens wird dem Knochenzementpulver wenigstens ein Röntgenopaker zugemischt. Beim Röntgenopaker kann es sich um einen fachüblichen Röntgenopaker handeln, bevorzugt in partikulärer Form. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen, wie Calciumcarbonat und Calciumsulfat besteht. Dabei sind Zirkoniumdioxid, Bariumsulfat, Calciumcarbonat und Calciumsulfat bevorzugt. Diese Röntgenopaker weisen vorzugsweise einen mittleren Teilchendurchmesser im Bereich von 10 nm bis 500 µm auf. Die Konzentration an zugemischtem Röntgenopaker, insbesondere die Konzentration an Zirkoniumdioxid, in dem Knochenzementpulver liegt bevorzugt in einem Bereich von 3 bis 30 Gew.-%, besonders bevorzugt in einem Bereich von 5,0 bis 20 Gew.-% liegen.

Gemäß einer weiteren bevorzugten Ausführungsform der Erfindung dem Knochenzementpulver wenigstens ein weiterer pharmazeutischen Wirkstoff zugemischt werden. Der wenigstens eine weitere pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum. Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht, besonders bevorzugt aus der Gruppe der Aminoglykosid-Antibiotika, Glykopeptid-Antibiotika, der Lincosamin-Antibiotike, der Lincosamid-Antbiotika, der Oxazolidinon-Antibiotika und der cyclischen Lipopeptide. Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Clindamycin, Ramoplanin, Metronidazol und Daptomycin sowie Salzen und Estern davon besteht. Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht. Das wenigstens eine Hormon ist vorzugsweise aus der Gruppe ausgewählt, die aus Serotonin, Somatotropin, Testosteron und Östrogen besteht. Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF)*,* dem *Bone Morphogenetic Protein* (BMP), Interleukin-1B, Interleukin 8 und dem Nervenwachstumsfaktor besteht. Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht. Das wenigstens eine Bisphosphonat ist vorzugsweise aus der Gruppe ausgewählt, die aus Zoledronat und Aledronat besteht.

Bevorzugt wird der oder werden die weiteren pharmazeutischen Wirkstoffe in einer Menge von 0,1 bis 15 Gew.-%, bevorzugt in einer Menge von 0,3 bis 12 Gew.% zugemischt, bezogen auf das Gesamtgewicht des Knochenzementpulvers.

Im Anschluss an das Vermischen der Knochenzementkomponenten wird das Knochenzementpulver sterilisiert. Verfahren zur Sterilisierung von polymerisierbaren Monomeren sind auf dem Gebiet der Medizinprodukte bekannt.

Wesentlich an dem erfindungsgemäßen Verfahren ist, dass der Wassergehalt der eingesetzten Rohstoffe die beschriebenen Grenzen nicht überschreitet und dass das Knochenzementpulver auf einen Wassergehalt von kleiner gleich 1,0 Gew.-% getrocknet wird.

Erfindungsgemäß wird das Knochenzementpulver vorteilhaft zunächst in einem Sterilpackmittel verpackt. Dabei sind sämtliche Packmittel geeignet, die den Anforderungen an medizinische und pharmazeutische Packmittel genügen und eine Sterilisation des Inhalts erlauben. Beispiele hierfür sind Verbundmaterialien aus Polyethylenfolie oder Polypropylenfolie und Tyrek® der Firma DuPont, einem schleuderverbundenen Fasermaterial aus hochdichtem Polyethylen.

Von den üblichen Sterilisationsverfahren werden vorliegend bevorzugt chemische Verbindungen zur Sterilisation von Medizinprodukten eingesetzt. Als für das vorliegende Verfahren besonders geeignet hat sich die Sterilisation mit Hilfe eines Gemisches aus Ethylenoxid, Wasserdampf und Kohlendioxid erwiesen.

Anschließend wird da Knochenzementpulver auf einen Wassergehalt von kleiner gleich 1,0 Gew.-% getrocknet. Die Trocknung des Knochenzementpulvers kann nach bekannten Verfahren durchgeführt werden. Die Trocknung kann zum Beispiel im Warmluftstrom oder bevorzugt im Vakuum erfolgen.

Nach der Trocknung wird das im Sterilpackmittel sterilisierte Knochenzementpulver in einem für Wasserdampf undurchlässigen weiteren Packmittel verpackt. Geeignete Packmittel sind metalldampfdichte Packmittel aus Polypropylen oder Polyethylen hoher Dichte, wie z.B. Verbundfolien mit einer Aluminiumschicht oder Blister. Denkbar sind aber auch feste Behälter z.B. aus Aluminium. Als wasserdampfundurchlässiges Packmittel bevorzugt sind Aluminiumverbundfolie und Aluminium-kaschiertes Papier.

Polymethylmethacrylat-Knochenzemente liegen in der Regel als Zweikomponentensysteme vor. Die erste Komponente ist gewöhnlich ein Knochenzementpulver. Das Knochenzementpulver wird auch als Polymethylmethacrylat-Knochenzementpulver bezeichnet. Die zweite Komponente enthält ein polymerisierbares Monomer, in der Regel Methylmethacrylat, und ist üblicherweise eine Flüssigkeit. Durch die Mischung der beiden Komponenten wird ein plastisch verformbarer Knochenzementteig erhalten, der nach einer gewissen Zeit aushärtet.

Mit dem erfindungsgemäßen Knochenzementpulver kann durch Vermischung mit üblicher Monomerflüssigkeit, die z.B. aus Methylmethacrylat, N,N-Dimethyl-p-toluidin, p-Hydrochinon zusammengesetzt ist, ein plastisch verformbarer Knochenzementteig hergestellt werden, der nach Aushärtung die Anforderungen der ISO 5833 hinsichtlich der Biegefestigkeit von mindestens 50 MPa, des Biegemoduls von mindestens 1800 MPa und der Druckfestigkeit von mindestens 70 MPa erfüllt.

Das erfindungsgemäße Polymethylmethacrylatzementpulver ist besonders als Bestandteil von Revisions-Polymethylmethacrylat-Knochenzementen, zur Herstellung von Spacern und zur Herstellung von implantierbaren lokalen Wirkstofffreisetzungssystemen geeignet.
Unter Revisions-Polymethylmethacrylat-Knochenzementen versteht man Polymethylmethacrylat-Knochenzemente, die zur dauerhaften Fixierung von Revisionsgelenkendoprothesen bestimmt sind, die im Rahmen der einzeitigen und auch der zweizeitigen septischen Revision von infizierten Gelenkendoprothesen verwendet werden. Unter dem Begriff Spacer werden temporäre Implantate verstanden, die im Rahmen der zweizeitigen septischen Revision von infizierten Gelenkendoprothesen als temporäre Platzhalter eingesetzt werden. Das Polymethylmethacrylat-Knochenzementpulver kann auch zur Herstellung von lokalen Wirkstofffreisetzungssystemen eingesetzt werden, wobei das Knochenzementpulver mit üblichen Gemischen aus Methylmethacrylat und einem tertiären Amin, z.B. N,N-Dimethyl-p-toluidin, vermischt wird, wobei ein selbsthärtender Zementteig entsteht, der in beliebige Form gegossen oder modelliert werden kann, wobei nach der Aushärtung durch radikalische Polymerisation mechanisch stabile Formkörper entstehen. Diese können im Rahmen der lokalen Antibiotika-Therapie verwendet werden. Die Wirkstofffreisetzungssysteme können kugelförmig, bohnenförmig, stabförmig ausgebildet sein. Es ist auch möglich, kugelförmige oder bohnenförmige Formkörper auf biokompatible Drähte aufzubringen.

Die Erfindung wird durch nachstehende Beispiele erläutert, die jedoch die Erfindung nicht einschränken.

### AUSFÜHRUNGSBEISPIELE

Die erfindungsgemäßen Pulver B1-4 wurden jeweils durch Vermahlen der Komponenten in einem dreidimensionalen Schüttelmischer (Turbula®-Mischer, Willy A. Bachofen AG Maschinenfabrik, Muttenz, Schweiz) hergestellt.

Für die erfindungsgemäßen Pulver wurde jeweils ein Gemisch aus 88,53 g Polymethylmethacrylat-co-methylacrylat (Wassergehalt 0,78 Gew-% bestimmt mit Karl-Fischer Titration), 10,00 g Zirkoniumdioxid und 1,47 g Dibenzoylperoxid (Dibenzoylperoxid phlegmatisiert mit 25 Gew.-% Wasser) hergestellt.

Den erfindungsgemäßen Pulvern B1-4 wurde außerdem Tromethamol-Fosfomycin der Firma Ecros (Spanien) in den in Tabelle 1 aufgeführten Mengen zugesetzt.

**Tabelle 1**

| Beispiel | Zusammensetzung des Knochenzementpulvers | | |
|---|---|---|---|
| | Wirkstoffreies Knochenzementpulver | Trometamol-fosfomycin* | Gentamicinsulfat ** |
| B1 | 40,0 g | 1,88 g | - |
| B2 | 40,0 g | 2,82 g | - |
| B3 | 40,0 g | 3,76 g | - |
| B4 | 40,0 g | 2,82g | 0,9 |

| | | | |
|---|---|---|---|
| *Aktivitätskoefizient Trometamol-Fosfomycin 533 **Aktivitätskoeffizient Gentamicinsulfat 571 | | | |

Die Knochenzementpulver der Beispiele B1-4 wurden anschließend mit einem Gemisch aus Ethylenoxid, Wasserdampf und Kohlendioxid sterilisiert. Danach wurden die Knochenzementpulver durch Vakuumeinwirkung auf einen Wassergehalt kleiner 1,0 % getrocknet. Die Knochenzementpulver der Beispiele B1-4 lagen nach der Trocknung als rieselfähige, nicht klumpende Pulver vor.

Die Bestimmung des Wassergehaltes der mit Ethylenoxid-sterilisierten und anschließend getrockneten Knochenzementpulver der Beispiele B1-4 wurde mit der Karl-Fischer Titrationsmethode vorgenommen. Dazu wurde ein Titrator Metrohm 802Tistand eingesetzt. Die Ergebnisse sind in Tabelle 2 aufgeführt.

**Tabelle 2**

| Beispiel | Wassergehalt Gew.-% |
|---|---|
| B1 | 0,53 |
| B2 | 0,51 |
| B3 | 0,52 |
| B4 | 0,87 |

Weiterhin wurden mit den sterilisierten, getrockneten Knochenzementpulvern und Verwendung von Palacos®-Monomerflüssigkeit der Firma Heraeus Medical GmbH, Wehrheim, Deutschland, die aus Methylmethacrylat, Hydrochinon, N,N-Dimethyl-p-toluidin und dem Farbstoff E141 zusammengesetzt ist, Formkörper zur Bestimmung der mechanischen Parameter nach ISO 5833 hergestellt. Die Zusammensetzungen der Knochenzemente sind in Tabelle 3 zu finden.

**Tabelle 3**

| | Zusammensetzung des Zementteigs | |
|---|---|---|
| Beispiel | Knochenzementpulver | Monomerflüssigkeit |
| B1 | 41,9 g | 20 ml |
| B2 | 42,8 g | 20 ml |
| B3 | 43,8 g | 20 ml |
| B4 | 43,7 g | 20 ml |

Aus den Pasten B1-4 wurden streifenförmige Prüfkörper mit den Abmessungen (75 mm x 10 mm x 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden dann 24 Stunden bei 23 ± 1 °C an der Luft gelagert. Danach wurden die 4-Punkt-Biegefestigkeit, das Biegemodul und die Druckfestigkeit der Prüfkörper mit einer Zwick-Universalprüfmaschine ermittelt. Die Ergebnisse sind in Tabelle 4 aufgeführt.

**Tabelle 4**

| Beispiel | 4-Punkt-Biegefestigkeit [MPa] | Biege-Modul [MPa] | DruckFestigkeit [MPa] |
|---|---|---|---|
| B1 | 70,5 ± 1,5 | 2949 ± 92 | 98,2 ± 2,4 |
| B2 | 73,1 ± 1,3 | 3211 ± 40 | 98,4 ± 1,6 |
| B3 | 65,2 ± 2,2 | 2923 ±88 | 90,1 ± 0,9 |
| B4 | 64,7 ± 1,2 | 2903 ± 53 | 98,3 ± 1,7 |

Die Prüfung der 4-Punkt-Biegefestigkeit, des Biegemoduls und der Druckfestigkeit der Formkörper aus den Pasten B 1-4 zeigt, dass die Anforderungen der ISO 5833 hinsichtlich der mechanischen Festigkeit erfüllt werden. Die ISO 5833 schreibt folgende Parameter vor: eine 4-Punkt-Biegefestigkeit von mindestens 50 MPa, ein Biegemodul von mindestens 1800 MPa und eine Druckfestigkeit von mindestens 70 MPa.

## Patentansprüche

1. Verfahren zur Herstellung eines antibiotischen Knochenzementpulvers, wobei in einem Schritt A) ein Knochenzementbasispulver mit einem Wassergehalt kleiner gleich 1,0 Gew.-% mit Trometamol-Fosfomycin zu einem Knochenzementpulver vermischt wird und in einem Schritt X) das Knochenzementpulver auf einen Wassergehalt von kleiner gleich 1,0 Gew.% getrocknet wird.

2. Verfahren nach Anspruch 1, **dadurch gekennzeichnet, dass** das
Knochenzementbasispulver ein Polymerpulver ist, wobei das Polymer ausgewählt ist aus der Gruppe bestehend aus Poly(methacryl-säuremethylester), Poly-(methacrylsäureethylester), Poly-(methylmeth-acrylsäurepropylester),
Poly(methacrylsäureisopropylester), Poly(methyl-methacrylat-co-methyl-acrylat), Poly(styrol-co-methyl-meth-acrylat) und einer Mischung aus mindestens zwei dieser Polymere.

3. Verfahren nach Anspruch 1 oder Anspruch 2, **dadurch gekennzeichnet, dass** in Schritt A) das Knochenzementbasispulver dem Knochenzementpulver in einer Menge von 70 bis 99,5 Gew.%, bevorzugt von 80,0 bis 94 Gew.-% zugemischt wird.

4. Verfahren nacheinem der vorangehenden Ansprüche, **dadurch gekennzeichnet, dass** in Schritt A) dem Knochenzementpulver ferner ein Röntgenopaker zugemischt wird.

5. Verfahren nach Anspruch 4, **dadurch gekennzeichnet, dass** der Röntgenopaker in einer Menge von 3 bis 30 Gew.-%, bevorzugt in einem Bereich von 5,0 bis 20 Gew.-% zugemischt wird.

6. Verfahren nach einem der vorhergehenden Ansprüche, **dadurch gekennzeichnet, dass** in Schritt A) dem Knochenzementpulver ein Initiator zugemischt wird.

7. Verfahren nach Anspruch 6, **dadurch gekennzeichnet, dass** es sich bei dem Initiator um ein Peroxid, insbesondere um mit Wasser phlegmatisiertes Dibenzoylperoxid mit einem Wassergehalt kleiner 28,0 Gew.-% handelt.

8. Verfahren nach Anspruch 6 oder 7, **dadurch gekennzeichnet, dass** der Initiator in einer Menge von 0,01 bis 10 Gew.-%, mehr bevorzugt in einer Menge von 0,2 bis 8 Gew.-% und noch mehr bevorzugt in einem Bereich von 0,5 bis 5 Gew.-%, jeweils bezogen auf das Gesamtgewicht des Knochenzementpulvers zugemischt wird.

9. Verfahren nach einem der vorangehenden Ansprüche, **dadurch gekennzeichnet, dass** in Schritt A) ein weiterer pharmazeutischen Wirkstoff zugemischt wird.

10. Verfahren nach Anspruch 9, **dadurch gekennzeichnet, dass** es sich bei dem pharmazeutischen Wirkstoff um ein Antibiotikum handelt, das einen Wassergehalt von maximal 15,0 Gew.% hat.

11. Verfahren nach Anspruch 9 oder 10, **dadurch gekennzeichnet, dass** die weiteren pharmazeutischen Wirkstoffe in einer Menge von 0,1 bis 15 Gew.-%, bevorzugt in einer Menge von 0, 3 bis 12 Gew.%, bezogen auf das Gesamtgewicht des Knochenzementpulvers, zugemischt werden.

12. Verfahren nach einem der vorangehenden Ansprüche, **dadurch gekennzeichnet, dass** nach dem Schritt A) und vor dem Schritt X) in einem Schritt E) das Knochenzementpulver sterilisiert wird.

13. Verfahren nach Anspruch 12, **dadurch gekennzeichnet, dass** das in Schritt A) hergestellte Knochenzementpulver vor der Sterilisation in Schritt E) in einem Schritt D) in einem Sterilpackmittel verpackt wird.

14. Knochenzementpulver hergestellt nach einem Verfahren gemäß einer der vorhergehenden Ansprüche, welches ein Knochenzementbasispulver und Trometamol-Fosfomycin enthält, wobei das Knochenzementpulver einen Wassergehalt von kleiner gleich 1,0 Gew.% hat.

15. Knochenzementpulver nach Anspruch 14, enthaltend
75,0-90,0 Gew.% mindestens eines partikulären Polymethylmethacrylats oder einse Polymethylmethycrylat-Copolymers, bezogen auf das Gesamtgewicht des Knochenzementpulvers
5,0-20,0 Gew.% eines partikulären Röntgenopakers, bezogen auf das Gesamtgewicht des Knochenzementpulvers
0,5-2,0 Gew.% mit Wasser phlegmatisiertes Dibenzoylperoxid ,bezogen auf das Gesamtgewicht des Knochenzementpulvers, mit einem Wassergehalt kleiner gleich 30 Gew.%, und
0,5-15,0 Gew.% Trometamol-Fosfomycin, bezogen auf das Gesamtgewicht des Knochenzementpulvers,
wobei der Gesamtwassergehalt des Knochenzementpulvers kleiner gleich 1,0 Gew.% ist.

16. Knochenzementpulver nach Anspruch 15, **dadurch gekennzeichnet, dass** außerdem ein Antibiotikum aus den Gruppen der Aminoglykosid-Antibiotika, der Glykopeptid-Antibiotika, der Licosamid-Antibiotika, der Oxazolidinon-Antibiotika enthalten ist, wobei das Antibiotikum einen Wassergehalt von maximal 15,0 Gew.-% hat.

17. Verwendung eines Knochenzementpulvers nach einem der Ansprüche 14 bis 16 als Bestandteil von Revisions-Polymethylmethacrylat-Knochenzementen, zur Herstellung von Spacern und zur Herstellung von implantierbaren lokalen Wirkstofffreisetzungssystemen.

## Claims

1. Method for the production of an antibiotic bone cement powder, whereby, in a step A), a bone cement base powder with a water content of less than or equal to 1.0% by weight is being mixed with trometamol-fosfomycin to form a bone cement powder and, in a step X), the bone cement powder is being dried to attain a water content of less than or equal to 1.0% by weight.

2. Method according to claim 1, **characterised in that** the bone cement base powder is a polymer powder, whereby the polymer is selected from the group consisting of poly(methacrylic acid methyl ester), poly(methacrylic acid ethyl ester), poly(methylmethacrylic acid propyl ester), poly(methacrylic acid isopropyl ester), poly(methyl-methacrylate-co-methyl acrylate), poly(styrene-co-methyl-methacrylate), and a mixture of at least two of said polymers.

3. Method according to claim 1 or claim 2, **characterised in that**, in step A), an amount ranging from 70 to 99.5% by weight, preferably ranging from 80.0 to 94% by weight, of the bone cement base powder is being admixed to the bone cement powder.

4. Method according to any one of the preceding claims, **characterised in that**, in step A), in addition, a radiopaquer is being admixed to the bone cement powder.

5. Method according to claim 4, **characterised in that** an amount from 3 to 30% by weight, preferably in a range of 5.0 to 20% by weight, of the radiopaquer is being admixed.

6. Method according to any one of the preceding claims, **characterised in that**, in step A), an initiator is being admixed to the bone cement powder.

7. Method according to claim 6, **characterised in that** the initiator is a peroxide, in particular water-phlegmatised dibenzoyl peroxide with a water content of less than 28.0% by weight.

8. Method according to claim 6 or 7, **characterised in that** an amount ranging from 0.01 to 10% by weight, more preferably an amount ranging from 0.2 to 8% by weight, and even more preferably an amount in the range of 0.5 to 5% by weight, each relative to the total weight of the bone cement powder, of the initiator is being admixed.

9. Method according to any one of the preceding claims, **characterised in that**, in step A), a further pharmaceutical agent is being admixed.

10. Method according to claim 9, **characterised in that** the pharmaceutical agent is an antibiotic that has a water content of maximally 15.0% by weight.

11. Method according to claim 9 or 10, **characterised in that** an amount ranging from 0.1 to 15% by weight, preferably an amount ranging from 0.3 to 12% by weight, relative to the total weight of the bone cement powder, of the further pharmaceutical agents is being admixed.

12. Method according to any one of the preceding claims, **characterised in that**, after step A) and before step X), the bone cement powder is being sterilised in a step E).

13. Method according to claim 12, **characterised in that** the bone cement powder produced in step A) is being packaged in sterile packaging means in a step D) prior to the sterilisation in step E).

14. Bone cement powder produced according to a method according to any one of the preceding claims, containing a bone cement base powder and trometamol-fosfomycin, whereby the bone cement powder has a water content of less than or equal to 1.0% by weight.

15. Bone cement powder according to claim 14, containing 75.0-90.0% by weight of at least one particulate polymethylmethacrylate or a polymethylmethacrylate copolymer, relative to the total weight of the bone cement powder,
5.0-20.0% by weight of a particulate radiopaquer, relative to the total weight of the bone cement powder,
0.5-2.0% by weight of water-phlegmatised dibenzoyl peroxide, relative to the total weight of the bone cement powder, with a water content of less than or equal to 30% by weight, and
0.5-15.0% by weight trometamol-fosfomycin, relative to the total weight of the bone cement powder,
whereby the total water content of the bone cement powder is less than or equal to 1.0% by weight.

16. Bone cement powder according to claim 15, **characterised in that** it contains, in addition, an antibiotic from the group of the aminoglycoside antibiotics, glycopeptide antibiotics, lincosamide antibiotics, oxazolidinone antibiotics, whereby the antibiotic has a water content of maximally 15.0% by weight.

17. Use of a bone cement powder according to any one of the claims 14 to 16 as a component of revision polymethylmethacrylate bone cements, for the production of spacers, and for the production of implantable local agent release systems.

## Revendications

1. Procédé de fabrication d'une poudre de ciment osseux antibiotique, dans lequel une poudre de base de ciment osseux avec une teneur en eau inférieure ou égale à 1,0 % en poids est mélangée dans une étape A) avec de la fosfomycine-trométamol en une poudre de ciment osseux et la poudre de ciment osseux est séchée dans une étape X) à une teneur en eau inférieure ou égale à 1,0 % en poids.

2. Procédé selon la revendication 1, **caractérisé en ce que** la poudre de base de ciment osseux est une poudre de polymère, dans lequel le polymère est sélectionné parmi le groupe constitué de l'ester méthylique d'acide poly(méthacrylique), l'ester éthylique d'acide poly(méthacrylique), l'ester propylique d'acide poly(méthyl-méthacrylique), l'ester isopropylique d'acide poly(méthacrylique), le poly(méthyl-méthacrylate-co-méthyl-acrylate), le poly(styrène-co-méthyl-méthacrylate) et un mélange d'au moins deux de ces polymères.

3. Procédé selon la revendication 1 ou la revendication 2, **caractérisé en ce que** la poudre de base de ciment osseux est ajoutée par mélange à l'étape A) à la poudre de ciment osseux en une quantité de 70 à 99,5 % en poids, de préférence de 80,0 à 94 % en poids.

4. Procédé selon une des revendications précédentes, **caractérisé en ce qu'**un opacifiant radiographique est en outre ajouté par mélange à la poudre de ciment osseux à l'étape A).

5. Procédé selon la revendication 4, **caractérisé en ce que** l'opacifiant radiographique est ajouté par mélange en une quantité de 3 à 30 % en poids, de préférence dans une plage de 5,0 à 20 % en poids.

6. Procédé selon une des revendications précédentes, **caractérisé en ce qu'**un initiateur est ajouté par mélange à la poudre de ciment osseux à l'étape A).

7. Procédé selon la revendication 6, **caractérisé en ce qu'**il s'agit pour l'initiateur d'un peroxyde, notamment de peroxyde de dibenzoyle flegmatisé avec de l'eau avec une teneur en eau inférieure à 28,0 % en poids.

8. Procédé selon la revendication 6 ou 7, **caractérisé en ce que** l'initiateur est ajouté par mélange en une quantité de 0,01 à 10 % en poids, de manière davantage préférée en une quantité de 0,2 à 8 % en poids et de manière encore davantage préférée dans une plage de 0,5 à 5 % en poids, à chaque fois par rapport au poids total de la poudre de ciment osseux.

9. Procédé selon une des revendications précédentes, **caractérisé en ce qu'**une autre substance active pharmaceutique est ajoutée par mélange à l'étape A).

10. Procédé selon la revendication 9, **caractérisé en ce qu'**il s'agit pour la substance active pharmaceutique d'un antibiotique qui a une teneur en eau de 15,0 % en poids maximum.

11. Procédé selon la revendication 9 ou 10, **caractérisé en ce que** les autres substances actives pharmaceutiques sont ajoutées par mélange en une quantité de 0,1 à 15 % en poids, de préférence en une quantité de 0,3 à 12 % en poids, par rapport au poids total de la poudre de ciment osseux.

12. Procédé selon une des revendications précédentes, **caractérisé en ce que** la poudre de ciment osseux est stérilisée dans une étape E) après l'étape A) et avant l'étape X).

13. Procédé selon la revendication 12, **caractérisé en ce que** la poudre de ciment osseux fabriquée à l'étape A) est emballée dans un emballage stérile dans une étape D) avant la stérilisation à l'étape E).

14. Poudre de ciment osseux fabriquée selon un procédé selon une des revendications précédentes, qui contient une poudre de base de ciment osseux et de la fosfomycine-trométamol, dans laquelle la poudre de ciment osseux a une teneur en eau inférieure ou égale à 1,0 % en poids.

15. Poudre de ciment osseux selon la revendication 14, contenant 75,0 à 90,0 % en poids d'au moins un polyméthylméthacrylate particulaire ou un copolymère de polyméthylméthacrylate, par rapport au poids total de la poudre de ciment osseux,
5,0 à 20,0 % en poids d'un opacifiant radiographique particulaire, par rapport au poids total de la poudre de ciment osseux,
0,5 à 2,0 % en poids de peroxyde de dibenzoyle flegmatisé avec de l'eau, par rapport au poids total de la poudre de ciment osseux, avec une teneur en eau inférieure ou égale à 30 % en poids, et
0,5 à 15,0 % en poids de fosfomycine-trométamol, par rapport au poids total de la poudre de ciment osseux,
dans laquelle la teneur en eau totale de la poudre de ciment osseux est inférieure ou égale à 1,0 % en poids.

16. Poudre de ciment osseux selon la revendication 15, **caractérisée en ce qu'**un antibiotique parmi les groupes des antibiotiques d'aminoglycoside, des antibiotiques de glycopeptide, des antibiotiques de licosamide, des antibiotiques d'oxazolidinone, est en outre contenu, dans laquelle l'antibiotique a une teneur en eau de 15,0 % en poids maximum.

17. Utilisation d'une poudre de ciment osseux selon une des revendications 14 à 16 en tant que constituant de ciments osseux de polyméthylméthacrylate de révision, pour la fabrication d'écarteurs et pour la fabrication de systèmes de libération d'ingrédient actif locaux implantables.
